# EUROPEAN PATENT APPLICATION

(11) **EP 3 795 122 A1**
(43) Date of publication of application: **24.03.2021**
(21) Application number: 19804177.4
(22) Date of filing: 27.03.2019
(51) Int. Cl.: A61F 5/455, A61F 5/44

(54) **REUSABLE MENSTRUAL CUP FOR EASY REMOVAL WITHOUT PAIN**

(30) Priority: 18.05.2018 KR 20180057072; 17.10.2018 KR 20180124000
(71) Applicant: Zamat Medical Co., Ltd., Seoul 07563 (KR)
(72) Inventor: IM, Kyung Ran, Seoul 08012 (KR)
(74) Representative: Shearman, James Ward
(86) International application number: PCT/KR2019/003571
(87) International publication number: WO 2019/221382

(57) **Abstract**

The present invention relates to a menstrual cup that can be reused by replacing only a container containing menstrual blood. The present invention uses a cover body made of a silicon material and penetrating vertically, and inside the cover body, a receiving cup is mounted, wherein the receiving cup is manufactured inexpensively by using a thin plastic material and includes an inner space for receiving menstrual blood therein, an inlet is formed on the upper surface thereof such that menstrual blood may flow into the inner space, and a protruding handle portion is formed such that the menstrual cup worn at the bottom end thereof can be easily separated from the inside a vagina and the cover body, and the body of the receiving cup has a restoring force automatically when pressed together with the cover body for facilitating wearing, and the receiving cup comprises longitudinal corrugated portions having space portions formed therebetween by concave portions and convex portions such that removal can be facilitated by releasing the vacuum inside the vagina at the time of removal after use. As such, it is possible to reuse the menstrual cup by replacing only the receiving cup in the cover body after use.

## Description

### [Technical Field]

The present invention relates to a menstrual cup, and more particularly, to a menstrual cup that is easily removed without causing pain by releasing the internal vacuum in a worn state, and reused by replacing only the container containing menstrual blood after use.

### [Background Art]

In general, women begin menstruation (menstruation) as the biggest change that occurs in the body during puberty. Most women use sanitary napkins to prevent discomfort of leaking menstrual blood to the outside for more hygienic and comfortable activities during menstruation.

Such a sanitary napkin, which is in the form of a pad, is attached to the inside of the panty for use by using attachment wings formed on both left and right sides thereof. The sanitary napkin is not only very inconvenient to wear for use, but also occur frequently in which menstrual blood leaks to the outside according to movement and bury in the panty when there is a large amount of menstruation or when wearing is wrong. In particular, a very difficult situation may occur when the user is out.

Therefore, recently, a tampon inserted into the vagina to improve the discomfort of a sanitary napkin in the form of a pad has been used.

Such a tampon, which is made of a stick-shaped absorbent material and inserted into the vagina to absorb menstrual blood, has the advantage of being very easy to wear and absorb, but recently, a problem has been raised that some users sometimes cause vomiting symptoms or shock syndrome due to toxicity.

Therefore, a menstrual cup made of a cup-shaped silicone material that is harmless to the human body and has good resilience has been disclosed, and such a menstrual cup can be easily inserted into the vagina, and prevents menstrual blood from leaking to the outside in a worn state, so that the menstrual cup's comfort is excellent even in everyday vigorous activities and life.

Meanwhile, since the silicone menstrual cup is made of silicone for reuse, the manufacturing cost is very expensive, and a vacuum is generated inside the vagina in the process of removing the menstrual cup inserted into the vagina after use, so that the menstrual cup is not easily removed, thereby causing pain. In particular, some menstrual blood contained in the menstrual cup that is forcibly discharged for removal is not only poured out due to compression, but also has troublesome problems such as a menstrual blood treatment process and a washing operation of the treated menstrual cup to reuse the menstrual cup. In addition, when the user is out, there are many inconveniences in processing and storage for carrying.

Therefore, although the menstrual cup made of silicon is expensive, it is mostly discarded after use due to various problems, which places a great burden on consumers.

### [Disclosure]

### [Technical Problem]

Therefore, to solve the problems described above, an object of the present invention is to provide a menstrual cup which is formed in a dual structure consisting of a reusable cover body made of a material harmless to the human body and having resilience and a receiving cup made of an inexpensive and resilient material for receiving menstrual blood, so that the menstrual cup is easy to put on and take out. In addition, when being taken out from inside the vagina, the menstrual cup is ventilated to release the vacuum so that the menstrual cup can be easily taken out without causing pain. After the menstrual cup is used, only the receiving cup can be easily separated, processed, and exchanged from the cover body, so that the cover body can be reused.

### [Technical Solution]

To achieve the objects, according to the present invention, there is provided a menstrual cup which includes a cover body made of a silicone material and penetrated vertically such that the cover body can be worn without irritation inside the sensitive vagina while having resilience, an inner space for containing menstrual blood therein, an inlet formed on an upper surface to allow menstrual blood to flow into the inner space, a protruding handle for separating the menstrual cup worn at a bottom end from an inside of a vagina or from the cover body, and a plastic receiving cup having a thin thickness, which is fitted into the inside of the cover body not to be arbitrarily separated from the cover body, wherein the receiving cup includes a body having strength while automatically having a restoring force when pressed together with the cover body, and formed with a vertical wrinkle portion formed with a space line between convex and concave so that air is vented when taken out in a worn state to release vacuum in the vagina, so that the menstrual cup can be easily removed and reused by replacing only the receiving cup from the cover body after use.

In addition, in order to prevent arbitrary separation of the receiving cup installed into the cover body, the receiving cup is configured in a form of an egg having narrow upper and lower ends and a convex body, and is tightly closed to an inner wall surface of the cover body, In addition, the receiving cup includes a support frame inserted into the lower end of the receiving cup, and a reinforcement rim formed on an opened lower end rim of the cover body to be latched to the support frame.

In addition, a space portion is formed at an inner side of an upper end rim bent to an upper surface of the body of the cover body, such that, when the handle of the receiving cup is pulled out, external air is circulated through the space line while being contracted by the wrinkle portion, thereby releasing the vacuum inside the vagina so that the worn menstrual cup may be easily taken out.

In addition, the upper surface of the receiving cup is formed to be inclined downward to the center where the inlet is formed so that menstrual blood naturally flows into the inner space through the inlet. The inclined upper surface reinforces the receiving cup to prevent distortion when the receiving cup is mounted or separated on or from the cover body, or inserted or taken out into or from the inside of the vagina.

In addition, the handle formed in the receiving cup extends from the inner space to form a space in which menstrual blood can be contained, a connection portion protruding toward the lower end of the receiving cup is concave, and a front end is convex, so that the handle is easily gripped without slipping, so the receiving cup is easy to separate from the cover body.

In addition, one or more locking frames are formed on the inner surface of the opened lower rim of the cover body to engage a finger or one or more ring portions are formed on the rim of the lower entrance, so that, when only the receiving cup is separated from the cover body and the cover body remains inside the vagina in the process of removing the menstrual cup worn inside the vagina using the handle of the receiving cup, the cover body may be easily taken out using the protrusion frame or the hook portion.

In addition, a close contact surface that is in close contact with the upper surface of the receiving cup is formed on the upper end rim of the cover body, so that it is possible to prevent menstrual blood from being randomly leaked into the gap between the contact surface of the cover body and the upper surface of the receiving cup while having a solid mounting structure.

Reinforcement rims are formed at uniform intervals on the lower side of the upper surface and the inner bottom surface of the receiving cup, so that the receiving cup is completely restored to its original state when folded and inserted for insertion into the vagina.

In addition, the inner space of the receiving cup is provided with an absorbent or cotton absorbing means compressed in the form of small beads that gradually swell by absorption, so that the liquid menstrual blood is absorbed by the absorbing unit to prevent menstrual blood from leaking to the outside.

### [Advantageous Effects]

Therefore, according to the present invention, the menstrual cup includes a cover body made of silicone and the receiving cup formed with a body having a wrinkled portion and made of soft plastic to receive menstrual blood, thereby providing an inexpensive manufacturing cost, as well as being fully contracted and restored, and making it easy to wear. When removing the worn menstrual cup, the vacuum state inside the vagina can be released so that the menstrual cup can be easily taken out without causing pain and can be easily used. In addition, by separating and discarding only the inexpensive receiving cup from the cover body after use, it is possible to reuse as well as to facilitate post-processing, providing economic benefits to both manufacturers and consumers.

### [Description of Drawings]

FIG. 1 is an exploded perspective view of a menstrual cup according to an embodiment of the present invention.

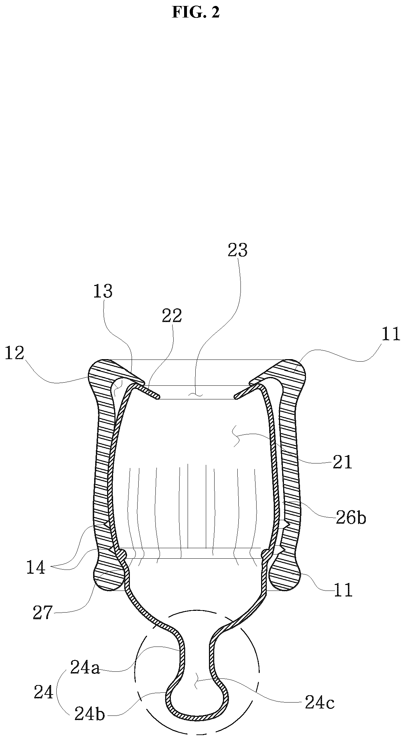

FIG. 2 is an assembled cross-sectional view of a menstrual cup according to the present invention.

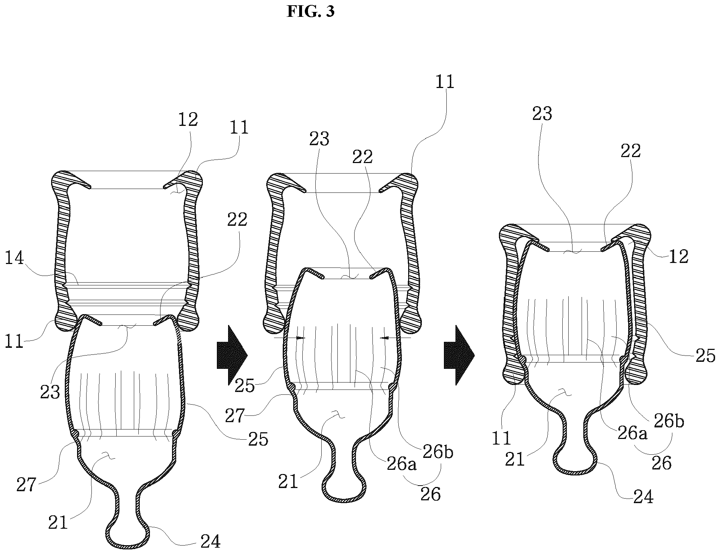

FIG. 3 is a cross-sectional view of a state in which a receiving cup is mounted on a cover body according to the present invention.

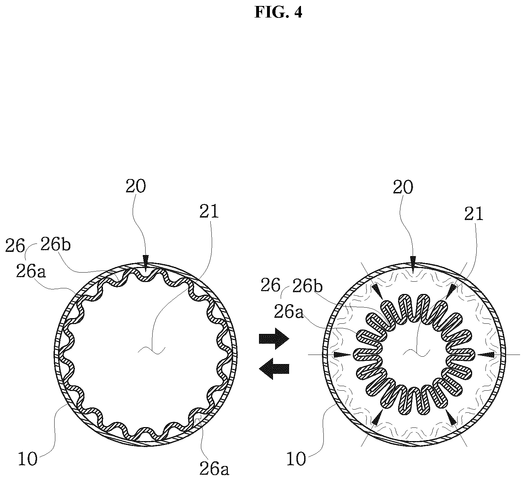

FIG. 4 is a cross-sectional view showing the restoring force of a menstrual cup according to the present invention.

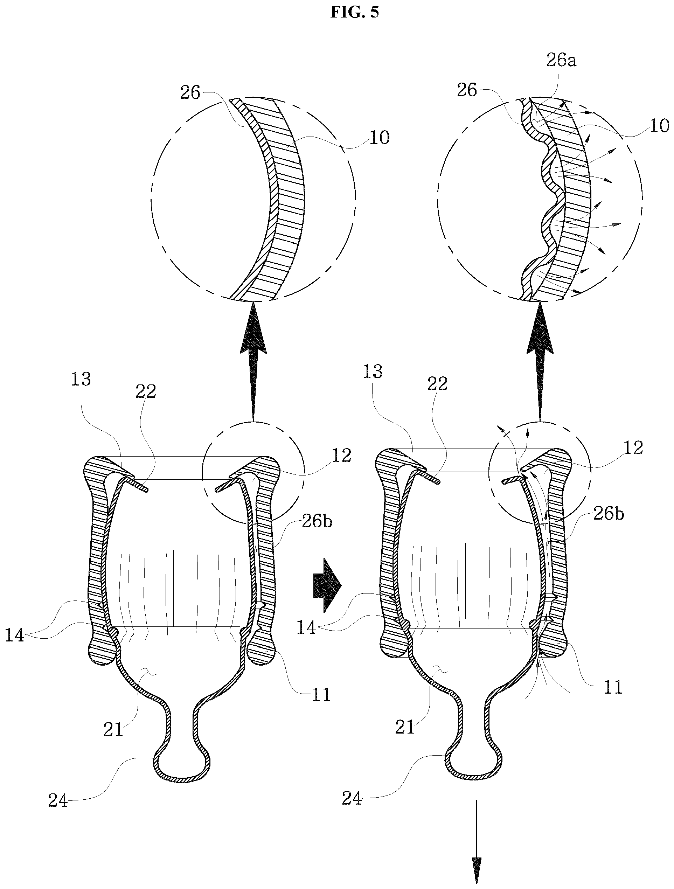

FIG. 5 is a cross-sectional view showing a vacuum release structure of the worn menstrual cup according to the present invention.

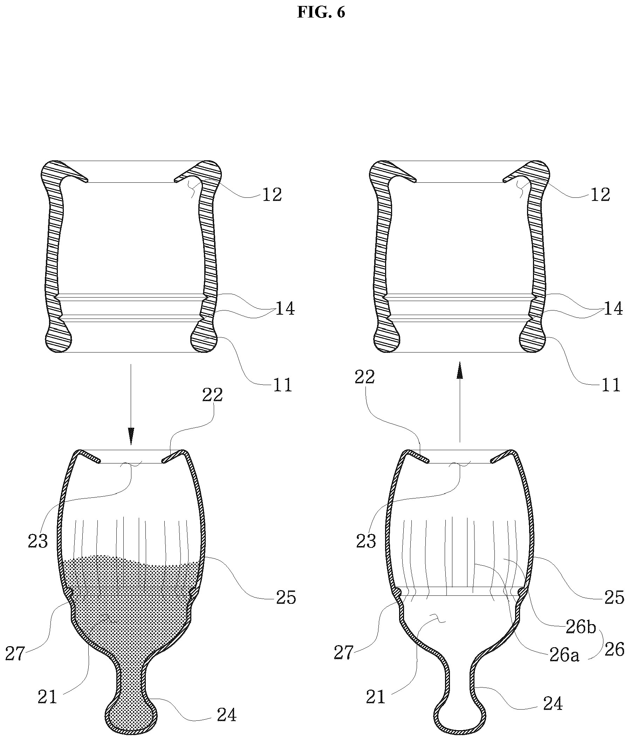

FIG. 6 is a diagram showing a state of reuse by replacing a receiving cup containing menstrual blood according to the present invention.

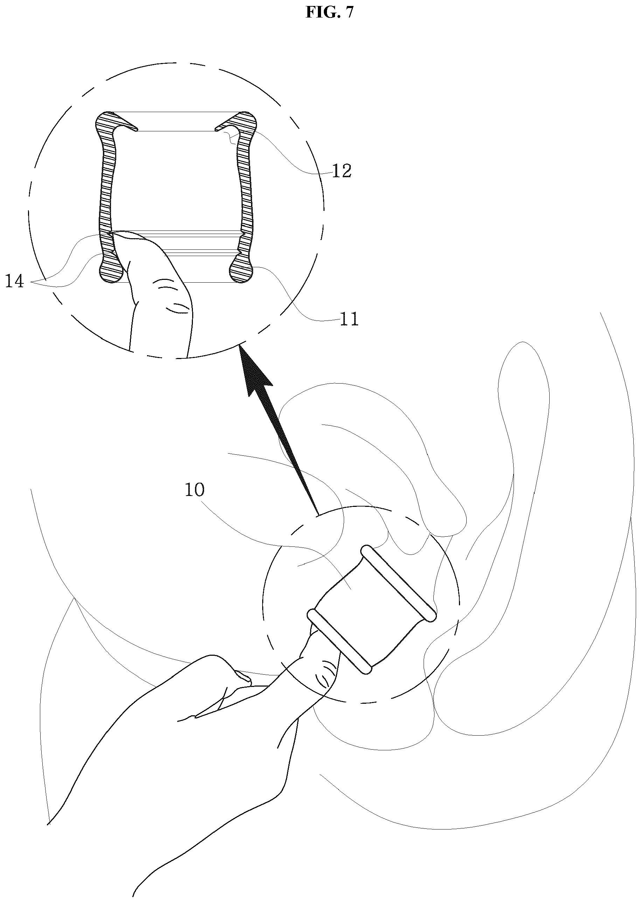

FIG. 7 is a diagram showing a state of taking out a cover body inside a vagina according to the present invention.

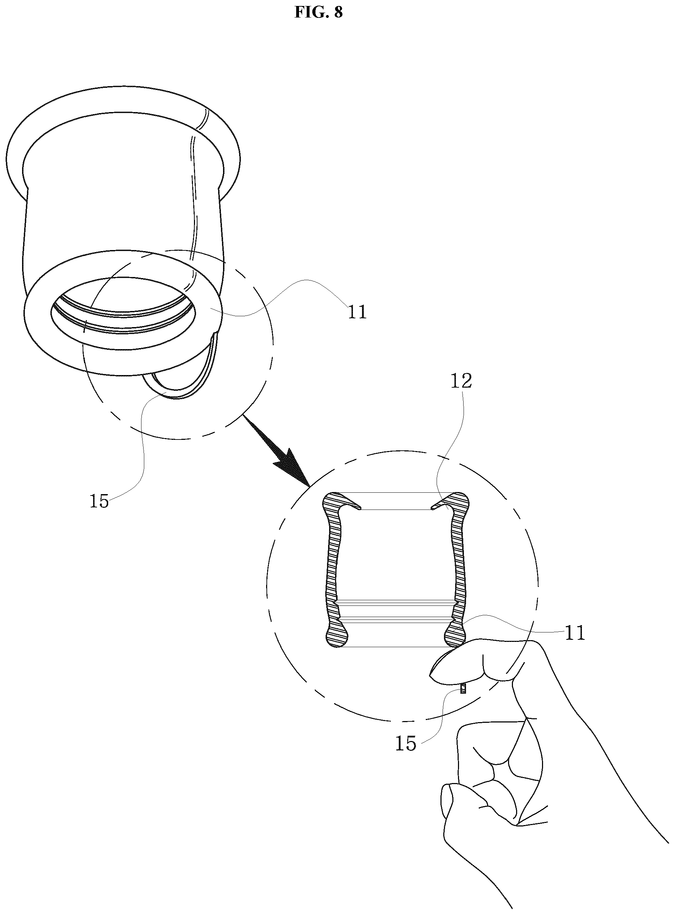

FIG. 8 is a diagram showing a state of taking out a cover body inside a vagina according to another embodiment of the present invention.

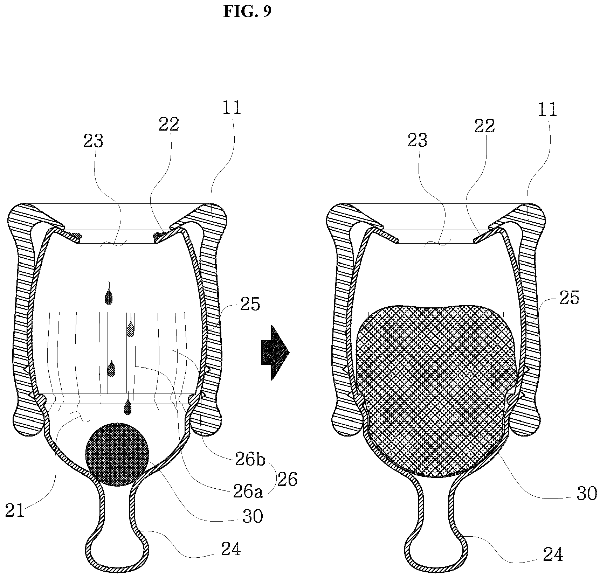

FIG. 9 is a view showing a state in which menstrual blood is absorbed by an absorption unit according to the present invention.

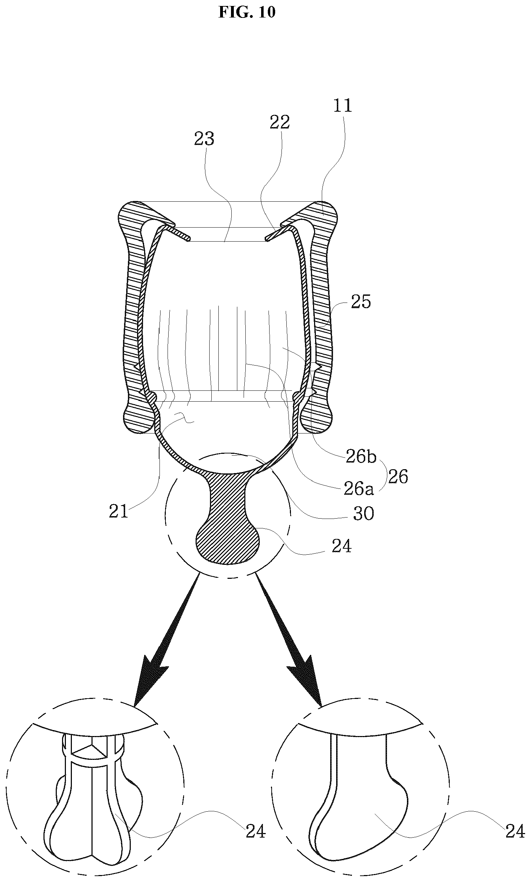

FIG. 10 is a view showing a handle according to another embodiment of the present invention.

### [Best Mode]

As the best mode according to an embodiment of the present invention, there is provided a menstrual cup which includes

a receiving cup made of a plastic material and installed inside the cover body to contain menstrual blood, wherein the menstrual cup is reused by replacing only the receiving cup in the cover body after use,

wherein the receiving cup includes an inner space for containing menstrual blood therein, an inlet formed on an upper surface to allow menstrual blood to flow into the inner space, a handle for separating the menstrual cup worn at a bottom end from an inside of a vagina or from the cover body, and a body is formed with a wrinkle portion having a convex portion in close contact with the cover body in a vertical direction and a concave groove portion that forms a gap between the cover body and the convex portion so that external air flows through the gap, and wherein, when the menstrual cup worn is taken out, the receiving cup is contracted and restored by the wrinkle portion to allow the menstrual cup to be easily taken out and an internal vacuum is released, thereby allowing the menstrual cup to be easily taken out without causing pain.

### [Mode for Invention]

Hereinafter, a menstrual cup according to an embodiment of the present invention will be described in detail with reference to the accompanying drawings.

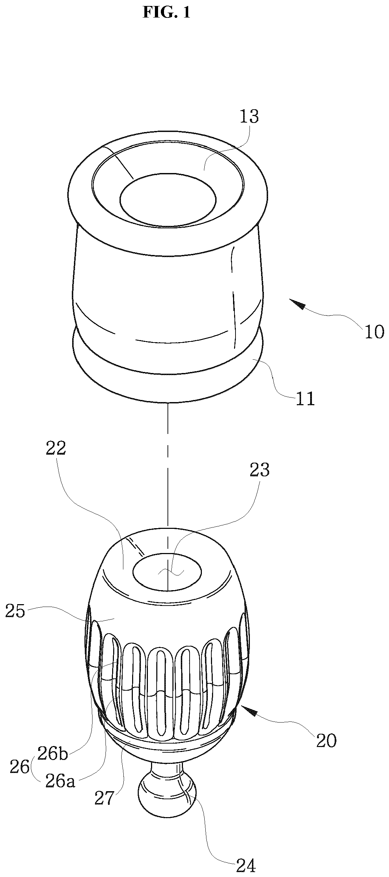

As shown in FIG. 1, a menstrual cup 1 includes a cover body 19 made of an elastic silicone material and a receiving cup 20 made of a plastic material to contain menstrual blood.

In this case, the cover body 10 is made of a cylindrical tube that penetrates vertically, and the receiving cup 20 is installed therein. Since the cover body 10 is made of a silicone material having a soft touch, the cover body 10 prevents a feeling of rejection when contacting the inside of the vagina made of sensitive skin to provide an easy fit and is harmless to the human body, providing safety at the same time.

In addition, the receiving cup 20 has an inner space 21 for containing menstrual blood therein, an inlet 23 formed in an upper surface 22 therein to allow menstrual blood to flow into the inner space 21, and a handle portion 24 having a predetermined length formed at a lower end to easily take out the inserted menstrual cup 1 from the vaginal or be separated from the cover body 10. The body 25 has a wrinkle portion 26 having convex and concave groove portions 26a and 26b in a vertical direction such that the receiving cup 20 made of the plastic material is contracted by pressing together with the cover body 10 for insertion into a vagina, and automatically restored in an inserted state. When the receiving cup 20 is contracted, a gap is formed by the concave groove portion 26b of the wrinkle portion 26 between the receiving cup 20 and the cover body 10, so that the vacuum state inside the vagina where the menstrual cup 1 is worn is released, thereby allowing the menstrual to be easily taken out.

That is, when the body 25 of the receiving cup 20 is pushed and pressed, the wrinkle portion 26 is tightly contracted by the concave groove portion 26b, and when the pressure is released in the contracted state, the body 25 is restored to the original state due to the restoration action so that the receiving cup 20 may be easily worn.

In addition, when the menstrual cup 1 worn inside a vagina is taken out, external air is introduced into the vagina by the concave groove portion 26a of the wrinkle portion 26 formed in the body 25 of the receiving cup 20 so that the vacuum state is released. Thus, the menstrual cup 1 worn inside the vagina may be easily taken out without causing pain.

Therefore, the menstrual cup 1 of the present invention may be easily worn and taken out inside the vagina, and the cover body 10 made of a silicone material may be reused only by washing. In addition, after only the receiving cup 20 containing the menstrual blood is separated from the cover body 10, the new receiving cup 20 is exchanged for reuse, so that it is possible to reuse the menstrual cup 1.

In particular, the receiving cup 20 is made of a PE-based soft plastic material having a thin thickness, but has a certain strength so that the receiving cup 20 may be contracted and restored while maintaining its shape without being crushed, and the cost may be cheaper than that of a conventional menstrual cup made of a silicon material. In addition, the post-treatment after use may be facilitated and the cover body 10 may be reusable after washing only by repeatedly replacing the receiving cup 20, thereby reducing the purchase burden on a consumer.

In this case, the upper surface 22 on which the inlet 23 of the receiving cup 20 is formed is preferably formed to be inclined downward so that menstrual blood flows naturally into the inner space 21 and is contained in the receiving cup 20. When the upper surface 22 of the receiving cup 20 is inclined so that the menstrual cup 1 is contracted by pressure to be inserted into the vagina, the upper surface 22, which is formed in a funnel shape together with the wrinkle portion 26 of the body 25, is easily restored to its original state by the restoration action.

However, the structure of the upper surface 22 does not limit the object of the present invention.

In this case, circular band-shaped reinforcement rims 11 are formed on the upper and lower rims of the cover body 10 having a vertically-perforated cylindrical shape, each of which has a diameter smaller than that of the body and a thickness thicker than that of the body, and the reinforcement rims 11 provide the reinforcing force to the cover body 10, thereby improving the restoration force for restoring the cover body 10 to the original state.

In addition, the receiving cup 20 has the body 25 having curved surface upper and lower surfaces. The upper surface 22 is formed to have a narrow width for insertion of the cover body 10. The body 25 is manufactured by brow molding in an egg shape, which is gradually convex on the upper surface 22 and gradually narrowed to the curved surface of a lower end.

The narrow upper end portion of the receiving cup 20 is inserted toward the opened lower end rim of the cover body 10, so that the receiving cup 20 is easily fitted by the elasticity of the cover body 10 and the contraction and restoring force of the wrinkle portion 26 of the receiving cup 20.

In this case, the receiving cup 20 installed by the adhesion of the cover body 10 is not arbitrarily separated, and when separated, the friction area is minimized by the wrinkle portion 26 formed on the body of the receiving cup 20, so that the receiving cup 20 is separated only by pulling the handle 24.

In addition, in order to prevent the receiving cup 20 from being arbitrarily separated from the cover body 10 in a state in which the receiving cup 20 is installed into the cover body 10, a support frame 27 is formed on the lower end of the receiving cup 20 to be latched to the reinforcement rim 11 formed on the lower end rim of the cover body 10, so that a reinforced installation structure is formed in which the reinforcement rim 11 supports the support frame 27 of the receiving cup 20 or is supported by latching, thereby preventing the receiving cup from being arbitrarily separated.

Therefore, in a state in which the menstrual cup 1 is inserted into the vagina while the cover body 10 is in close contact with the body 25 of the receiving cup 20, only the receiving cup is prevented from being separated from the cover body 10.

In addition, a close contact surface 13, which extends inwardly to be in close contact with the upper end rim of the mounted receiving cup 20 or be overlapped with the upper surface 22, is further formed on the reinforcing rim 11 formed on the upper end rim of the cover body 10 to be in close contact, such that the gap formed by wrinkle portion 26 between the cover body 10 and the receiving cup 20 is blocked to prevent leakage of menstrual blood while reinforcing the mounting state of the cover body 10 and the receiving cup 20 more firmly, and menstrual blood is induced to flow only into the inner space 21 of the receiving cup 20. When the receiving cup 20 is pulled through the handle 24 to take out the menstrual cup, the contact surface 13 is separated and the gap of the wrinkle portion 26 is exposed, so that external air is introduced to release the vacuum.

In this case, by forming a space portion 12 on the inner side of the body and the bent upper surface of the reinforcing frame 11 formed on the opened upper end rim of the cover body 10, the upper outer wall surface of the receiving cup 20 installed inside the cover body 10 is not in close contact but is mounted in a spaced state by the space portion 12. In this state, when the handle 24 formed at the lower end of the receiving cup 20 is pulled, the receiving cup 20 is contracted by the wrinkle portion 26, and a gap due to the concave groove 26b is generated between the receiving cup 20 and the cover body 10 in close contact with the receiving cup 20 due to the concave groove portion 26b of the wrinkle portion 26, so that air is introduced into the space portion 12 to release the vacuum inside the vagina. Thus, the worn menstrual cup can be easily taken out without causing pain.

In addition, while the worn menstrual cup 1 is removed, when only the receiving cup 20 is separated from the cover body 10 and the cover body 10 remains inside the vagina, in order to easily remove the cover body 10, a locking rim 14 is formed on the inner side surface of the opened lower end rim of the cover body 10 so that the finger can be caught.

In addition, as another embodiment for taking out the cover body 10 remaining inside the vagina by separating only the receiving cup 20, one or more ring portions 15 are formed on the opened lower rim surface of the cover body 10, so that the cover body 10 can be easily taken out by using the ring portion 15 with a finger.

In addition, the handle 24 formed on the receiving cup 20 is formed to protrude outward from the lower end of the body 25, and in consideration that the receiving cup 20 is manufactured by brow molding, the auxiliary space 24c is formed inside so as to communicate with the received inner space 21 of the receiving cup 20 in which menstrual blood is contained such that the menstrual blood is partially contained in the auxiliary space 24c, thereby allowing the filled state to be checked and increasing the containing space.

As still another embodiment of the handle 24, the handle 24 is formed by crossing a thin plate or two plates in a cross shape. The handle 24 includes a concave connecting portion 24a for facilitating pulling and a finger gripping portion 24b formed to be convex on the front end of the connecting portion 24a.

An absorption unit 30, such as a compressed absorbent in the form of a small roll or bead, or cotton is rolled into a bead shape, in which menstrual blood is gradually swollen by absorption, is further provided in the inner space 21 of the receiving cup 20, such that the menstrual blood contained in the receiving cup 20 is solidified to prevent leakage, as well as facilitate the post-treatment of the receiving cup 20.

The absorption unit 30 is manufactured by compression molding in a roll or bead shape by inserting a nonwoven fabric made of 100% natural pulp material or rayon material into a mold of a certain shape.

Accordingly, the liquid menstrual blood is absorbed by the absorption unit 30 while being accommodated in the inner space 21 of the receiving cup 20, and the absorption unit 30 gradually swells to solidify the liquid menstrual blood, so that it is possible to prevent leakage when taking out from the vagina or separating the receiving cup 20 from the cover body 10. In particular, when the separated receiving cup 20 is discarded, menstrual blood does not leak to the outside of the receiving cup 20, thereby preventing environmental pollution.

In the present invention, the material of the absorbing unit 30 is described as a nonwoven fabric made of natural pulp or rayon, but the material is proposed as one embodiment and various materials may be used.

Although the present invention has been described with reference to a number of illustrative embodiments thereof and drawings, various modifications and variations are possible within the scope of the technical spirit of the present invention and the claims to be described below by those of ordinary skill in the art.

## Claims

1. A menstrual cup that is easily removed and reused without causing pain, the menstrual cup comprising:
a cylindrical cover body (10) made of an elastic silicone material and penetrated vertically; and
a receiving cup (20) made of a plastic material and installed inside the cover body (10) to contain menstrual blood,
wherein the menstrual cup (1) is reused by replacing only the receiving cup (20) in the cover body (10) after use,
wherein the receiving cup (20) includes an inner space (21) for containing menstrual blood therein, an inlet (23) formed on an upper surface (22) to allow menstrual blood to flow into the inner space (21), a handle (24) for separating the menstrual cup (1) worn at a bottom end from an inside of a vagina or from the cover body (10), and a body (25) is formed with a wrinkle portion (26) having a convex portion (26a) in close contact with the cover body (10) in a vertical direction and a concave groove portion (26b) that forms a gap between the cover body (10) and the convex portion (26a) so that external air flows through the gap, and
wherein, when the menstrual cup (20) worn is taken out, the receiving cup (20) is contracted and restored by the wrinkle portion (26) to allow the menstrual cup to be easily taken out and an internal vacuum is released, thereby allowing the menstrual cup to be easily taken out without causing pain.

2. The menstrual cup of claim 1, wherein the cover body (10) is formed with reinforcement rims (11) in upper and lower end rims thereof to reinforce restoring force,
wherein the receiving cup (20) is configured in a form of an egg having narrow upper and lower ends and a convex body, and is tightly closed to an inner wall surface of the cover body by an elastic force of the cover body (20), and
wherein a support frame (27) is formed on a lower end of the receiving cup (20) and is recessed inward so as to be latched with the reinforcement rim (11) formed on an opened lower end rim of the cover body (10) such that the receiving cup (20) is prevented from being arbitrarily separated from the cover body (10).

3. The menstrual cup of claim 2, wherein a close contact surface (13) covering an upper surface (22) of the receiving cup (20) is further formed on the reinforcement rim (11) formed on the upper end rim of the cover body (10) such that the gap formed by the wrinkle portion (26) between the cover body (10) and the receiving cup (20) is blocked to prevent leakage of menstrual blood.

4. The menstrual cup of claim 3, wherein a space portion (12) is formed at an inner side of the reinforcement rim (11) formed in the opened upper end rim of the cover body (11) while being spaced apart from an outside surface of the receiving cup (20), and
wherein, when the handle (24) of the receiving cup (20) is pulled, contraction is made by the wrinkle portion (26) to release a close contact of the close contact surface (13), and air is introduced into the space portion (12) through the gap formed by the concave groove portion (26b) of the wrinkle portion (26) to release vacuum inside the vagina, thereby allowing the receiving cup (20) to be easily taken out.

5. The menstrual cup of claim 1, wherein the handle (24) formed on the receiving cup (20) has a concave connection portion (24a) and a convex finger gripping portion (24b) formed on a front end of the connection portion (24a) to facilitate a pulling operation for taking out the receiving cup 20.

6. The menstrual cup of claim 1, wherein an auxiliary space (24) communicating with the inner space (21) of the receiving cup (20) is formed inside the handle (24) including the connection portion (24a) and the finger gripping portion (24b).

7. The menstrual cup of claim 1, wherein a latch rim (14) is formed in an inner surface of the opened lower end rim of the cover body (10), and
wherein, when only the receiving cup (20) is separated from the cover body (10) while the menstrual cup (20) worn inside the vagina is taken out, the cover body (10) remaining inside the vagina is easily taken out by using the latch rim (14).

8. The menstrual cup of claim 1, wherein one or more ring portions (15) are formed on the opened lower end rim of the cover body 10, and wherein, when only the receiving cup (20) is separated from the cover body (10) while the menstrual cup (20) worn in the vagina is taken out, the cover body (10) remaining in the vagina is easily taken out by using the ring portion (15).

9. The menstrual cup of claim 1, further comprising:
an absorbent (30) provided in the inner space (21) of the receiving cup (20) and compressed in a small bead shape, the absorbent (30) gradually swelling by absorption of menstrual blood.
